# EUROPÄISCHE PATENTANMELDUNG

(11) **EP 3 868 694 A1**
(43) Veröffentlichungstag der Anmeldung: **25.08.2021**
(21) Anmeldenummer: 21156260.8
(22) Anmeldetag: 10.02.2021
(51) Int. Cl.: B65H 29/24, A61F 13/15

(54) **DREHVORRICHTUNG FÜR EINE FERTIGUNGSANLAGE FÜR HYGIENEPRODUKTE**

(30) Priorität: 18.02.2020 DE 102020104275
(71) Anmelder: Bicma Hygiene Technologie GmbH, 56727 Mayen (DE)
(72) Erfinder: Spurzem, Heinrich, 56727 Mayen (DE)
(74) Vertreter: Gottschald Patentanwälte Partnerschaft mbB

(57) **Zusammenfassung**

Die Erfindung betrifft eine Drehvorrichtung (1) für eine Fertigungsanlage (2) für Hygieneprodukte, insbesondere Erwachsenen- oder Babywindeln, wobei die Drehvorrichtung eine um eine geometrische Trommeldrehachse (11) rotierende Trommel (12) und mindestens einen radial von der Trommeldrehachse (11) beabstandeten und mit der Trommel (12) gekoppelten Teller (14) aufweist, der eine Saugfläche (15) zum Transport von Hygienematerialstücken (16) umfasst, die das jeweilige Hygienematerialstück (16) während des Transports mittels eines Unterdrucks hält, wobei der Teller (14) eine radial zur Trommeldrehachse (11) verlaufende geometrische Hochachse (17) aufweist, das Hygienematerialstück (16) mit einer ersten Ausrichtung um die Hochachse (17) in einem ersten Transferbereich (18) aufnimmt und das Hygienematerialstück (16) entlang einer in Umfangsrichtung um die Trommeldrehachse (11) verlaufenden Soll-Transferbahn (19) von dem ersten Transferbereich (18) zu einem zweiten Transferbereich (20) transportiert, wobei die Saugfläche (15) mit dem Hygienematerialstück (16) während des Transports um die Hochachse (17) gedreht wird, wobei der Teller (14) das Hygienematerialstück (16) in dem zweiten Transferbereich (20) mit einer von der ersten Ausrichtung verschiedenen zweiten Ausrichtung um die Hochachse (17) abgibt. Es wird vorgeschlagen, dass der Teller (14) während der Aufnahme und/oder Abgabe eine nicht-lineare Ausgleichsbewegung relativ zur Trommel (12) mit radialem Bewegungsanteil durchführt.

## Beschreibung

Die Erfindung betrifft eine Drehvorrichtung für eine Fertigungsanlage für Hygieneprodukte, insbesondere Erwachsenen- oder Babywindeln, gemäß dem Oberbegriff von Anspruch 1 sowie eine Fertigungsanlage für Hygieneprodukte als solche gemäß Anspruch 14.

Unter Hygieneprodukten werden körpernah eingesetzte Saugkörper verstanden, insbesondere Erwachsenen- oder Babywindeln, Inkontinenz-Vorlagen und -Pants, Damenbinden, Slipeinlagen oder dergleichen. Hygieneprodukte haben zumeist gemein, dass sie einen flüssigkeitsabsorbierenden Kern als Hygienematerial aufweisen, der an einem Trägermaterial befestigt ist. Das Trägermaterial kann insbesondere durch eine körperseitige Materiallage (Top Sheet) und eine körperferne Materiallage (Back Sheet) ausgebildet sein. Daran können seitliche Befestigungselemente (Ohren) angesetzt sein, die zum Befestigen des Hygieneprodukts am Körper dienen.

Fertigungsanlagen für Hygieneprodukte haben üblicherweise eine Maschinenlaufrichtung, entlang der sowohl das Hygienematerial als auch das Trägermaterial geführt wird. Wenn das Hygienematerial und das Trägermaterial verbunden werden sollen, muss das Hygienematerial jedoch üblicherweise um 90° gedreht werden, um entsprechend quer zu dem Trägermaterial platziert zu werden. Dafür wird bei bekannten Fertigungsanlagen das Hygienematerial geschnitten und von einer Drehvorrichtung gedreht.

Bei einer bekannten Drehvorrichtung (WO 2019/215547 A1) wird das Schneiden des Hygienematerials ebenfalls auf der Drehvorrichtung erledigt. Dies hat den Nachteil, dass es nicht ohne weiteres möglich ist, mit ein und derselben Drehvorrichtung verschiedene Formate, also Größen, der Hygieneprodukte zu produzieren. Bei anderen bekannten Fertigungsanlagen wird daher vor der Drehvorrichtung geschnitten. Problematisch ist jedoch, dass bei der Übergabe der geschnittenen Hygienematerialstücke auf die Drehvorrichtung und von der Drehvorrichtung Verschiebungen und Verwerfungen auftreten können. Bekannte Drehvorrichtungen weisen zur Aufnahme der Hygienematerialstücke gewölbte Teller auf, die an eine Transferbahn der üblicherweise rotierenden Drehvorrichtung angepasst sind. Da diese Teller jedoch gedreht werden, ist eine flächige Übergabe sowohl bei der Aufnahme als auch bei der Abgabe der Hygienematerialstücke so nicht zu erreichen.

Bei einer weiteren bekannten Drehvorrichtung (US 2016/0376109 A1) erfolgt eine lineare Ausgleichsbewegung. Dies ist jedoch zum einen konstruktiv aufwändig, da hohe Toleranzen eingehalten werden müssen, und zum anderen ist die Anpassung an die kreisförmigen Transferbahnen der Fertigungsanlage nicht möglich.

Der Erfindung liegt das Problem zugrunde, die bekannten Drehvorrichtungen derart auszugestalten und weiterzubilden, dass unter Beibehaltung der Möglichkeit, verschiedene Formate zu produzieren, eine erhöhte Präzision bei der Übergabe erreicht wird.

Das obige Problem wird bei einer Drehvorrichtung gemäß dem Oberbegriff von Anspruch 1 durch die Merkmale des kennzeichnenden Teils von Anspruch 1 gelöst.

Grundsätzlich geht die Erfindung von einer Drehvorrichtung mit einer um eine geometrische Trommeldrehachse rotierenden Trommel und mindestens einem radial von der Trommeldrehachse beabstandeten und mit der Trommel gekoppelten Teller aus. Der Teller umfasst eine Saugfläche zum Transport von Hygienematerialstücken, die das jeweilige Hygienematerialstück während des Transports mittels eines Unterdrucks hält. Der Teller weist weiterhin eine radial zur Trommeldrehachse verlaufende geometrische Hochachse auf. Der Teller nimmt das Hygienematerialstück mit einer ersten Ausrichtung um die Hochachse in einem ersten Transferbereich auf und transportiert das Hygienematerialstück entlang einer in Umfangsrichtung um die Trommeldrehachse verlaufenden Soll-Transferbahn von dem ersten Transferbereich zu einem zweiten Transferbereich, wobei die Saugfläche mit dem Hygienematerialstück während des Transports um die Hochachse gedreht wird. Der Teller gibt das Hygienematerialstück in dem zweiten Transferbereich mit einer von der ersten Ausrichtung verschiedenen zweiten Ausrichtung um die Hochachse ab.

Wesentlich ist die grundsätzliche Überlegung, dass der Teller mittels einer Ausgleichsbewegung aktiv an die Übergabe angepasst werden kann. Durch diese Ausgleichsbewegung ist nicht mehr allein die Form des Tellers ausschlaggebend für den Kontakt zwischen Teller und Hygienematerialstück während der Übergabe.Dadurch können auch drehbare Teller das Hygienematerialstück präzise übergeben, ohne durch die beschränkten Möglichkeiten der Ausgestaltung der Oberfläche der Saugfläche begrenzt zu sein.

Im Einzelnen wird vorgeschlagen, dass der Teller während der Aufnahme und/oder Abgabe eine nicht-lineare Ausgleichsbewegung relativ zur Trommel mit radialem Bewegungsanteil durchführt.

Bei der Ausgestaltung gemäß Anspruch 2 weist die Ausgleichsbewegung des Tellers zusätzlich einen Bewegungsanteil in Umfangsrichtung um die Trommeldrehachse auf, wodurch komplexere Ausgleichsbewegungen ermöglicht werden. Diese können genauer auf die Geschwindigkeit der Drehvorrichtung und eines weiteren Teils der Fertigungsanlage im Übrigen, der das Hygienematerialstück aufnimmt oder abgibt, und die geometrischen Gegebenheiten der Drehvorrichtung und des aufnehmenden bzw. abgebenden Teils der Fertigungsanlage abgestimmt sein.

Für eine insbesondere mechanisch einfache Umsetzung kann gemäß Anspruch 3 vorgesehen sein, dass der Teller derart mit der Trommel gekoppelt ist, dass der Fortschritt der Ausgleichsbewegung von einem Drehwinkel der Trommel um die Trommeldrehachse abhängig ist.

Eine für die Gegebenheiten der Übergabe bei üblichen Drehvorrichtungen besonders geeignete Ausgestaltung gibt Anspruch 4 an, wonach der Teller mit der Trommel ein Koppelgetriebe bildet.

Insbesondere zur Ansteuerung des Koppelgetriebes kann bei einer Ausgestaltung gemäß Anspruch 5 vorgesehen sein, dass der Teller mittels eines Führungsgliedes mit einer Kurvenscheibe gekoppelt ist. Dadurch wird eine konstruktiv nicht besonders aufwendige Möglichkeit angegeben, die Ausgleichsbewegung durchzuführen.

Anspruch 6 gibt eine weitere bevorzugte und relativ einfach umzusetzende Ausgestaltung der Ausgleichsbewegung an.

Bei der Ausgestaltung gemäß Anspruch 7 erfolgt die Aufnahme und/oder die Abgabe des Hygienematerialstücks vollflächig und/oder mit konstanter Geschwindigkeit. So kann erreicht werden, dass gegenüber bekannten Drehvorrichtungen deutlich weniger Verschiebungen und Verwerfungen des Hygienematerialstücks auftreten, wodurch die Produktionsgenauigkeit der Fertigungsanlage steigt.

In einer besonders bevorzugten Ausgestaltung ist die Saugfläche des Tellers entlang seiner ersten und/oder zweiten Ausrichtung plan. Durch das Weglassen der gebogenen Saugflächen des Tellers kann bei beliebiger Ausrichtung eine vollflächige Übergabe erreicht werden.

Bei einer Ausgestaltung gemäß Anspruch 9 verläuft ein Teil der Saugfläche, der sich in dem ersten und/oder zweiten Transferbereich befindet, aufgrund der Ausgleichsbewegung während der Aufnahme und/oder Abgabe radial innerhalb der Soll-Transferbahn. Dies kann vorzugsweise außerhalb des ersten und/oder zweiten Transferbereichs nicht der Fall sein.

Um einen den Sog erzeugenden Unterdruck der Saugfläche genau dort zu aktivieren, wo dieser gebraucht wird, kann vorgesehen sein, dass die Saugfläche gemäß Anspruch 10 mehrere Saugsegmente umfasst. Diese Saugsegmente können einzeln und/oder in Gruppen ansteuerbar sein, wodurch erreicht werden kann, dass das Hygienematerialstück nicht von Saugsegmenten angesaugt wird, mit denen es noch nicht oder nicht mehr in Kontakt treten soll. Weiterhin ist dies hinsichtlich der Energieeffizienz vorteilhaft.

Die Ausgestaltungen gemäß Anspruch 11 und 12 betreffen die bevorzugten Ausgestaltungen der Trommel, der Trommeldrehachse und der Leitung des Unterdrucks. Gemäß Anspruch 13 kann die Drehvorrichtung mehrere gleichartige Teller aufweisen.

Nach einer weiteren Lehre gemäß Anspruch 14, der eigenständige Bedeutung zukommt, wird eine Fertigungsanlage für Hygieneprodukte mit einer vorschlagsgemäßen Drehvorrichtung beansprucht. Auf alle Ausführungen zu der vorschlagsgemäßen Drehvorrichtung darf verwiesen werden.

Die Ansprüche 15 und 16 geben bevorzugte Ausgestaltungen der Fertigungsanlage an, mit denen die vorschlagsgemäße Drehvorrichtung besonders vorteilhaft nutzbar ist.

Im Folgenden wird die Erfindung anhand einer lediglich ein Ausführungsbeispiel darstellenden Zeichnung näher erläutert. In der Zeichnung zeigt
- Fig. 1: die vorschlagsgemäße Drehvorrichtung in einer dreidimensionalen Ansicht von außen,
- Fig. 2: schematisch die Fertigungsanlage inklusive des Ablaufs einer Fertigung,
- Fig. 3: das Innenleben der vorschlagsgemäßen Drehvorrichtung und
- Fig. 4: den Ablauf der Ausgleichsbewegung beispielhaft in vergrößerter Form.

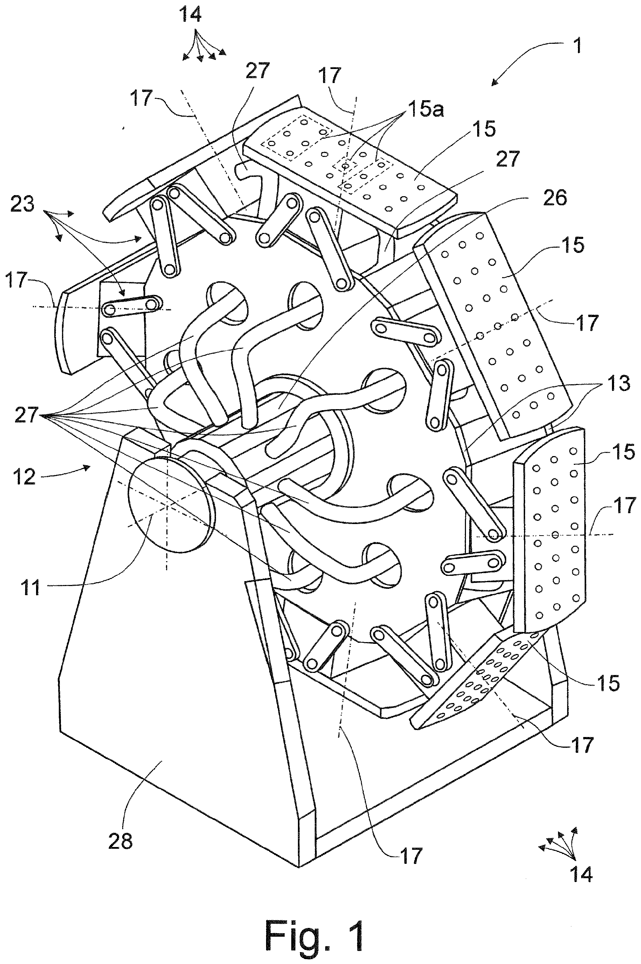

Fig. 1 zeigt die vorschlagsgemäße Drehvorrichtung 1 für eine Fertigungsanlage 2 für Hygieneprodukte, insbesondere Erwachsenen- oder Babywindeln. Bezüglich anderer möglicher Hygieneprodukte wird auf den einleitenden Teil der Beschreibung verwiesen.

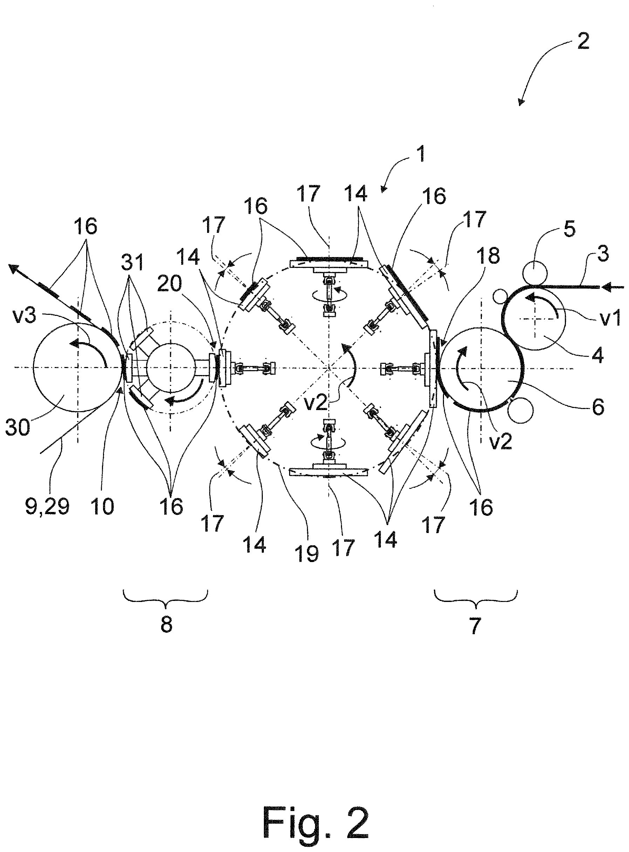

Fig. 2 zeigt schematisch den Aufbau einer bevorzugten Fertigungsanlage 2. Eine Hygienematerial-Bahn 3 wird der Fertigungsanlage 2 von rechts in Fig. 2 zugeführt und trifft dort auf eine erste Transportrolle 4 mit einem Querverschluss 5. Das Hygienematerial ist hier und vorzugsweise flächig und/oder als Saugmaterial ausgestaltet.

Diese erste Transportrolle 4 kann im Betrieb dabei eine erste Geschwindigkeit v1 aufweisen. Von dort kann die Hygienematerial-Bahn 3 an eine mit einer Geschwindigkeit v2 rotierende Schneidrolle 6 einer Schneideinheit 7 übergeben werden. Die Schneideinheit 7 weist ein Schneidgerät, beispielsweise ein Schneidmesser auf, das die Hygienematerial-Bahn 3 schneidet. Durch den aufgrund der unterschiedlichen Geschwindigkeiten v1 und v2 zwischen der ersten Rolle 4 und der Schneidrolle 6 entstehenden Schlupf wird das geschnittene Hygienematerial gleichzeitig vereinzelt. Sodann wird es von der noch zu erläuternden Drehvorrichtung 1 weiter in Fig. 2 nach links übertragen und dabei gedreht, von einer Repitch-Einheit 8, also einer Geschwindigkeits-Änderungs-Einheit, aufgenommen und auf eine Geschwindigkeit v3 beschleunigt und mit dem Trägermaterial 9 an einem "Marriage Point" 10 zusammengeführt. Der Grund dafür, dass das geschnittene Hygienematerial gedreht wird, ist üblicherweise, dass die Hygienematerial-Bahn 3 und das Trägermaterial 9 in einer Maschinenrichtung entlang der Fertigungsanlage 2 parallel verlaufen, jedoch quer zueinander zusammengeführt werden sollen.

In Fig. 1 ist die vorschlagsgemäße Drehvorrichtung 1 in einer Außenansicht dargestellt. Die Drehvorrichtung 1 weist eine um eine geometrische Trommeldrehachse 11 rotierende Trommel 12 auf. Der Begriff "Trommel" ist hier weit zu verstehen, im einfachsten Fall kann es sich bei der Trommel 12 um eine Scheibe 13 handeln. Ein Mantel ist bei der Trommel 12 nicht zwingend notwendig, kann aber grundsätzlich vorgesehen sein. Im einfachsten Fall dient die Trommel 12 auch lediglich der mechanischen Befestigung und Führung und muss entsprechend keine spezifischen Eigenschaften außer denen eines Trägers aufweisen. Hier und vorzugsweise ist die Trommel 12 endlosdrehend.

Die Drehvorrichtung 1 weist mindestens einen radial von der Trommeldrehachse 11 beabstandeten und mit der Trommel 12 gekoppelten Teller 14 auf. Hier und vorzugsweise weist die Drehvorrichtung 1 mehr als einen derartigen Teller 14 auf. Im Ausführungsbeispiel weist die Drehvorrichtung 1 acht Teller 14 auf. Im Folgenden beziehen sich die meisten Ausführungen der Einfachheit halber nur auf einen Teller 14. Hier und vorzugsweise sind die Teller 14 jedoch gleichartig. Alle Ausführungen zu dem einen Teller 14 gelten daher für die anderen Teller 14 entsprechend.

Im Folgenden werden einige Merkmale der Drehvorrichtung 1 mit dem Begriff "radial" und mit dem Begriff "Umfangsrichtung" beschrieben. Diese beziehen sich immer auf die Trommeldrehachse 11.

Der Teller 14 und die Trommel 12 sind bezüglich der Rotation um die Trommeldrehachse 11 bewegungsgekoppelt, so dass der Teller 14 mit der Trommel 12 um die Trommeldrehachse 11 rotiert. Hier und vorzugsweise sind der Teller 14 und die Trommel 12 ohne Schlupf bezüglich der Rotation um die Trommeldrehachse 11 bewegungsgekoppelt.

Der Teller 14 umfasst eine Saugfläche 15 zum Transport von Hygienematerialstücken 16. Die Saugfläche 15 hält das jeweilige Hygienematerialstück 16 während des Transports mittels eines Unterdrucks. So kann das Hygienematerialstück 16 auch quer oder senkrecht zur Schwerkraftrichtung transportiert werden. Hier und vorzugsweise wurde das Hygienematerialstück 16 wie bereits beschrieben von der Fertigungsanlage 2 mit der Schneideinheit 7 aus der Hygienematerial-Bahn 3 geschnitten.

Der Teller 14 weist eine radial zur Trommeldrehachse 11 verlaufende geometrische Hochachse 17 auf. Diese Hochachse 17 ist bezüglich der Saugfläche 15 des Tellers 14 definiert und kann, wie noch erläutert wird, insbesondere bezüglich der Trommeldrehachse 11 kippbar sein.

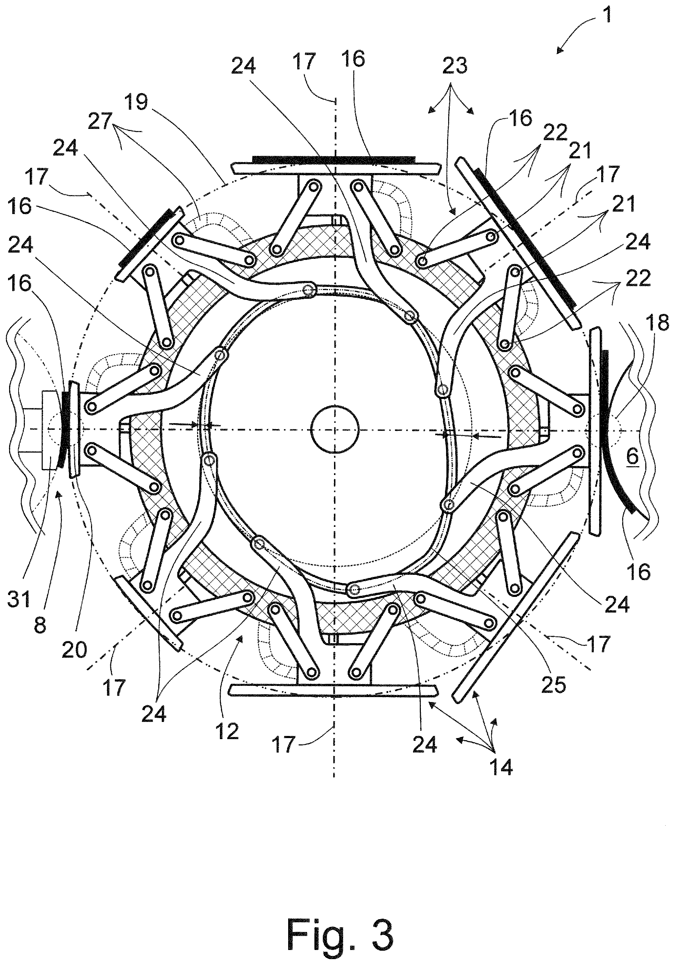

Im Betrieb nimmt der Teller 14 das Hygienematerialstück 16 mit einer ersten Ausrichtung um die Hochachse 17 in einem ersten Transferbereich 18 auf. Der Teller 14 transportiert das Hygienematerialstück 16 entlang einer in Umfangsrichtung um die Trommeldrehachse 11 verlaufenden Soll-Transferbahn 19 von dem ersten Transferbereich 18 zu einem zweiten Transferbereich 20. Dies lässt sich am besten der Darstellung in Fig. 3 entnehmen. Die Soll-Transferbahn 19 ist insbesondere kreisförmig und gibt, bezogen auf den Mittelpunkt des Hygienematerialstücks 16, an, wo das Hygienematerialstück 16 entlang transportiert wird. Hier und vorzugsweise ist die Soll-Transferbahn 19 durch den maximal radial vorhanden Platz im ersten Transferbereich 18 und im zweiten Transferbereich 20 definiert. Der erste Transferbereich 18 und/oder der zweite Transferbereich 20 sind hier und vorzugsweise linienförmig bzw. im Querschnitt wie in Fig. 2 gezeigt sogar nahezu punktförmig. Definiert sind die Transferbereiche 18, 20 als die Bereiche, in denen sich das Hygienematerialstück 16 bei der Übergabe befindet. Da diese Bereiche durch Toleranzen nicht bei jedem Hygienematerialstück 16 identisch sind, nutzt nicht jedes Hygienematerialstück 16 den vollen Transferbereich 18, 20 bei seiner Übergabe.

Die Saugfläche 15 mit dem Hygienematerialstück 16 wird während des Transports um die Hochachse 17 gedreht. Hier und vorzugsweise erfolgt diese Drehung um 90°. Hier und vorzugsweise erfolgt die Drehung außerdem zeitlich und örtlich während des Transports und insbesondere von einer Ausrichtung längs zu der Maschinenlaufrichtung der Fertigungsanlage 2 zu einer Ausrichtung quer zur Maschinenlaufrichtung der Fertigungsanlage 2. Der Teller 14 gibt das Hygienematerialstück 16 in dem zweiten Transferbereich 20 mit einer von der ersten Ausrichtung verschiedenen zweiten Ausrichtung um die Hochachse 17 gedreht, hier und vorzugsweise um 90° um die Hochachse 17 gedreht, ab.

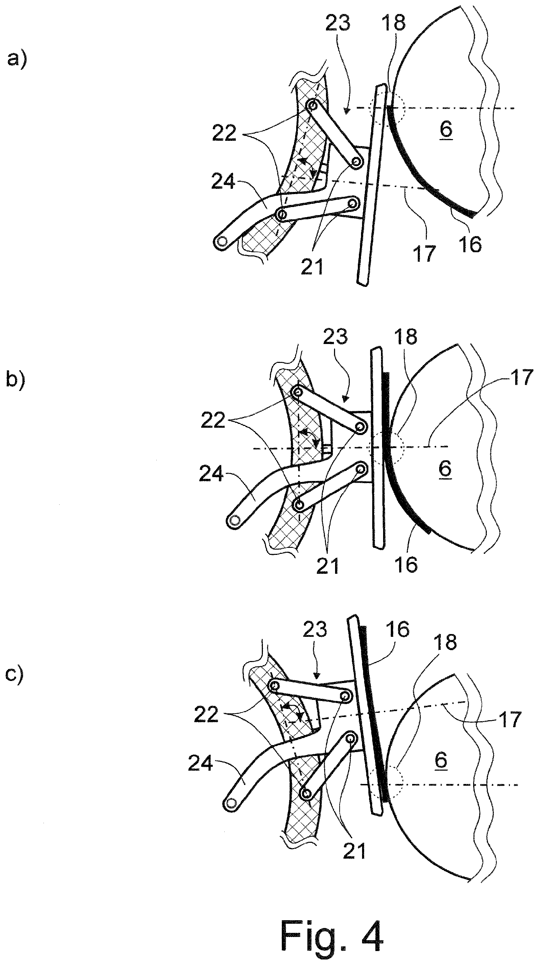

Vorschlagsgemäß führt der Teller 14 während der Aufnahme und/oder Abgabe eine nicht-lineare Ausgleichsbewegung relativ zur Trommel 12 mit radialem Bewegungsanteil durch (siehe insbesondere Fig. 4). Der Begriff "nicht-linear" bedeutet hier, dass die Ausgleichsbewegung als solche, also ohne Betrachtung der gleichzeitigen Rotation der Trommel, keine rein lineare Translation ist. Die Saugfläche 15 wird somit nicht rein linear in eine Richtung verschoben. Dabei ist ein Vorteil, dass eine komplexere Bewegung konstruktiv einfach umgesetzt werden kann und gleichzeitig eine bessere Anpassung an die vorliegenden zumeist kreisförmigen Bewegungsbahnen ermöglicht. Dabei kann durch die Inkaufnahme der komplexeren Bewegung die jeweilige Übergabe des Hygienematerialstücks 16 hinsichtlich diverser Faktoren optimiert werden.

Zum einen kann die Ausgleichsbewegung an eine Soll-Transferbahn einer abgebenden oder aufnehmenden Einheit, hier also der Schneideinheit 6 oder der Repitch-Einheit 8, angepasst sein.

Eine geometrische Rotationsachse der Soll-Transferbahn der abgebenden bzw. aufnehmenden Einheit wird üblicherweise außerhalb der Soll-Transferbahn der Drehvorrichtung 1 liegen. Daher ist es vorzugsweise so, dass die Ausgleichsbewegung eine Rotation um eine geometrische Rotationsachse umfasst und dass diese geometrische Rotationsachse außerhalb der Soll-Transferbahn der Drehvorrichtung 1 liegt. Besonders bevorzugt kann die geometrische Rotationsachse der Ausgleichsbewegung der geometrischen Rotationsachse der abgebenden oder aufnehmenden Einheit, insbesondere der Schneideinheit 6 oder der Repitch-Einheit 8, entsprechen.

Zum anderen kann zusätzlich oder alternativ eine Anpassung der Geschwindigkeit des Hygienematerialstücks 16 erfolgen, wenn die abgebende und die aufnehmende Einheit nicht die gleiche Geschwindigkeit wie die Drehvorrichtung 1 aufweist.

Auch kann gleichzeitig der Übernahmewinkel zwischen der Saugfläche 15 und dem Hygienematerialstück 16 angepasst werden.

Da Rotationsachsen zudem teilweise konstruktiv einfacher verwirklicht werden können, als Linearachsen, können diese Vorteile mit konstruktiven Vorteilen einhergehen.

Hier und vorzugsweise weist die Ausgleichsbewegung mindestens zwei radial gegenläufige Anteile auf. Dabei bewegt sich eine Seite der Saugfläche 15 radial nach innen und die andere Seite gleichzeitig radial nach außen.

Wie in den Figuren ersichtlich ist, erreicht der Teller 14 bzw. die Saugfläche 15 den ersten Transferbereich 18 von unten in eine Richtung gekippt und verlässt ihn oben in die andere Richtung gekippt. Dadurch kann der Teller 14 durch eine Art Nickbewegung an den Transferbereich 18, 20 angepasst werden. Auch im zweiten Transferbereich 20 ist die Ausgleichsbewegung in Fig. 3 dargestellt. Aufgrund der Drehung der Saugfläche 15 und der entsprechend kleineren Proportionen fällt sie dort jedoch entsprechend weniger ausladend aus. Hier und vorzugsweise führt der gesamte Teller 14 die Ausgleichsbewegung durch, es ist jedoch genauso vorteilhaft denkbar, dass nur ein Teil des Tellers 14, insbesondere die Saugfläche 15, die Ausgleichsbewegung durchführt.

Hier und vorzugsweise weist die Ausgleichsbewegung des Tellers 14 zusätzlich einen Bewegungsanteil in Umfangsrichtung um die Trommeldrehachse 11 auf. Zusätzlich oder alternativ kann die Ausgleichsbewegung eine Rotationsbewegung des Tellers 14 um eine erste geometrische Rotationsachse 21 umfassen.

Vorzugsweise umfasst die Ausgleichsbewegung zusätzlich eine Rotation der ersten Rotationsachse 21 um eine zweite Rotationsachse 22. Dies ist in den Fig. 3 und 4 veranschaulicht, wobei die erste Rotationsachse 21 und die zweite Rotationsachse 22 der Einfachheit halber nur bei einem Teller 14 mit Bezugszeichen versehen sind. Weiter vorzugsweise kann die Ausgleichsbewegung eine weitere Rotation um eine weitere erste Rotationsachse 21 und eine weitere Rotation der weiteren ersten Rotationsachse 21 um eine weitere zweite Rotationsachse 22 umfassen. Diese Rotationen können dabei zumindest teilweise gleichzeitig oder sequentiell ablaufen. Die erste und die zweite Rotationsachse 21, 22 sind vorzugsweise parallel, jedoch nicht koaxial, zur Trommeldrehachse 11 und/oder zueinander. Mit dem Begriff "Rotation" ist immer eine Rotation mit einem Freiheitsgrad um eine geometrische Rotationsachse gemeint. Der Bewegungsanteil der Ausgleichsbewegung in Umfangsrichtung versteht sich relativ zur Trommel 12, das heißt er weicht von der bereits vorhandenen Rotation der Trommel 12 ab.

Hier und vorzugsweise ist die Ausgleichsbewegung eine kontinuierliche Bewegung, die somit weder ein Stoppen noch einen abrupten Richtungswechsel umfasst. Nach Beendigung der Ausgleichsbewegung kann jedoch eine Rückstellung mit Richtungswechsel erfolgen.

Es kann vorgesehen sein, dass der Teller 14 derart mit der Trommel 12 gekoppelt ist, dass der Fortschritt der Ausgleichsbewegung, insbesondere nur, von einem Drehwinkel der Trommel 12 um die Trommeldrehachse 11 abhängig ist. Diese Kopplung ist hier und vorzugsweise mechanisch. Hier und vorzugsweise bedeutet dies ebenfalls, dass der Fortschritt der Ausgleichsbewegung von dem Drehwinkel des Tellers 14 um die Trommeldrehachse 11 abhängig ist. Dieser Drehwinkel ist grundsätzlich abhängig vom jeweiligen Teller 14. Es ist hier und vorzugsweise vorgesehen, dass jeder Teller 14 bei dem entsprechenden Drehwinkel dieselbe Ausgleichsbewegung durchführt.

Im Folgenden wird die Realisierung der Durchführung der Ausgleichsbewegung beschrieben, die ebenfalls am besten der Darstellung in Fig. 3 zu entnehmen ist. Hier und vorzugsweise bildet der Teller 14 mit der Trommel 12 ein Koppelgetriebe 23. Dem Begriff "Koppelgetriebe" liegt dabei ausdrücklich die in der Mechanik geläufige Definition zugrunde. Das Koppelgetriebe 23 ist hier und vorzugsweise ein viergliedriges Koppelgetriebe 23, insbesondere eine Viergelenkkette. Das Koppelgetriebe 23 kann ein ebenes Koppelgetriebe 23 sein, dessen Ebene dann vorzugsweise winkelig, insbesondere rechtwinkelig, zur Trommeldrehachse 11 ausgerichtet ist. Bei einem ebenen Koppelgetriebe sind die Achsen der Gelenke des Koppelgetriebes parallel zueinander ausgerichtet, sodass die Bewegungen des Koppelgetriebes in einer Ebene stattfinden. Wie dargestellt bildet der Teller 14 vorzugsweise ein Glied des Koppelgetriebes 23 und ist mit zwei weiteren Gliedern mit der Trommel 12 verbunden, die ebenfalls ein Glied des Koppelgetriebes 23 bildet. Hier und vorzugsweise rotiert der Teil des Tellers 14, der das Glied des Koppelgetriebes 23 bildet, nicht mit der Saugfläche 15 um die Hochachse 17.

Insbesondere zur Ansteuerung des Koppelgetriebes 23 kann der Teller 14 mittels eines Führungsgliedes 24 mit einer Kurvenscheibe 25, zu der der Teller 14 verlagerbar ist, gekoppelt sein. Die Ausgleichsbewegung wird dann durch die Kopplung des Tellers 14 über das von der Kurvenscheibe 25 geführte Führungsglied 24 mit der Kurvenscheibe 25 verursacht. Alternativ wäre jedoch auch beispielsweise eine passive, externe Führung der Ausgleichsbewegung denkbar. Hier und vorzugsweise ist die Kurvenscheibe 25 um die Trommeldrehachse 11 angeordnet. Die Kurvenscheibe 25 kann das Führungsglied 24 ein- oder zweiseitig führen, entsprechend kann das Führungsglied 24, insbesondere federbeaufschlagt, aufliegen oder in einer Führung der Kurvenscheibe 25, wie in Fig. 3 gezeigt, laufen. In Fig. 3 sind auch die unterschiedlichen Abstände der Führungsbahn der Kurvenscheibe 25 von einer idealen Kreisbahn bei Aufnahme und Abgabe des Hygienematerialstücks 16 eingezeichnet, die durch die rechteckige, jedoch nicht quadratische, Form der Hygienematerialstücke 16 und der Saugfläche 15 in Kombination mit der Drehung der Saugfläche 15 zustande kommen.

Genauso vorteilhaft wäre beispielsweise auch eine Ansteuerung des Tellers 14 mittels eines Kurbeltriebs oder andersartig denkbar. Hier und vorzugsweise rotiert die Kurvenscheibe 25 nicht mit der Trommel 12 um die Trommeldrehachse 11, sondern steht insbesondere still.

In einem nicht dargestellten Ausführungsbeispiel kann die Ausgleichsbewegung eine Rotation des Tellers 14 um eine geometrische Rotationsachse sein, die vorzugsweise parallel, jedoch nicht koaxial, zur Trommeldrehachse 11 verläuft. Diese Variante würde dann mit einer Änderung der Geschwindigkeit des Hygienematerialstücks 16 einhergehen.

Wie in Fig. 3 ersichtlich, kann das Führungsglied 24 starr mit dem Teller 14 gekoppelt sein. Alternativ ist beispielsweise und ebenso bevorzugt eine Abwandlung denkbar, bei der das Führungsglied 24 mittels eines Gelenks mit dem Teller 14 und/oder der Kurvenscheibe 25, dort insbesondere verschiebbar, gekoppelt ist. In noch einer Variante könnte das Führungslied 24 auch ein Glied des Koppelgetriebes 23 oder mit diesem verbunden sein.

Hier und vorzugsweise erfolgt die Aufnahme und/oder die Abgabe des Hygienematerialstücks 16 vollflächig mit einem konstanten Übergabeabstand. Der Übergabeabstand bezieht sich dabei auf den Abstand zwischen dem jeweils aufnehmenden bzw. abgebenden Teil des Tellers 14 und der Schneidrolle 6 oder der Repitch-Einheit 8 oder einem anderen denkbaren aufnehmenden oder abgebenden Bauteil der Fertigungsanlage 2 im Übrigen. Vollflächig bedeutet hier, dass das Hygienematerialstück 16 zwar stückweise, jedoch mit linienförmigem Kontakt mit dem Teller 14 übergeben wird. Der konstante Übergabeabstand ist im Wesentlichen konstant und schwankt maximal um 5%, vorzugsweise maximal um 1%, weiter vorzugsweise maximal um 0,5%.

Zusätzlich oder alternativ bleibt eine Geschwindigkeit des Hygienematerialstücks 16 während der Aufnahme und/oder Abgabe des Hygienematerialstücks 16 konstant und schwankt maximal um 5%, vorzugsweise maximal um 1%, weiter vorzugsweise maximal um 0,5%. Dabei ist eine Umlenkung des Hygienematerialstücks 16 vorgesehen, sowohl das aufnehmende als auch das abgebende Element weisen dabei jedoch die gleiche Geschwindigkeit auf.

In der gezeigten und insofern besonders bevorzugten Ausführungsform ist die Saugfläche 15 des Tellers 14 entlang seiner ersten und/oder zweiten Ausrichtung plan.

Hier und vorzugsweise verbleibt ein in dem ersten Transferbereich 18 befindlicher Teil der Saugfläche 15 des Tellers 14 aufgrund der Ausgleichsbewegung während der Aufnahme radial innerhalb der Soll-Transferbahn 19. Zusätzlich oder alternativ kann ein in dem zweiten Transferbereich 20 befindlicher Teil der Saugfläche 15 des Tellers 14 aufgrund der Ausgleichsbewegung während der Abgabe radial innerhalb der Soll-Transferbahn 19 verbleiben. Weiter zusätzlich oder alternativ kann die Saugfläche 15 entlang der ersten und/oder zweiten Ausrichtung nicht entlang der Soll-Transferbahn 19 verlaufen, insbesondere derart, dass die Saugfläche 15 in dem ersten und/oder zweiten Transferbereich 18, 20 nur aufgrund der Ausgleichsbewegung innerhalb des jeweiligen Transferbereichs 18, 20 verbleibt. In der Darstellung in Fig. 3 ist dies dadurch erkennbar, dass, von unten nach oben gesehen auf der rechten Seite die Saugfläche 15 mit dem bei einer Bewegung im Gegenuhrzeigersinn vorderen Ende in den Transferbereich 18 eintaucht und dann über eine Kippbewegung über das Koppelgetriebe 23 nach oben derart kippt, dass am Ende das hintere Ende im Transferbereich 18 innerhalb der Soll-Transferbahn 19 ist. Hier und vorzugsweise wird eine ähnliche Ausgleichsbewegung auch im zweiten Transferbereich 20 durchgeführt.

Damit das Hygienematerialstück 16 während der Übergabe nicht durch den Unterdruck der Saugfläche 15 abgelenkt wird, kann vorgesehen sein, dass die Saugfläche 15 mehrere Saugsegmente 15a umfasst, die vorzugsweise einzeln ansteuerbar sind. Die Ansteuerung der Saugsegmente 15a erfolgt hier und vorzugsweise derart, dass die Saugsegmente 15a abhängig von dem Drehwinkel der Trommel 12 und/oder einem Fortschritt der Ausgleichsbewegung angesteuert werden. Diese Ansteuerung kann elektronisch jedoch prinzipiell auch mechanisch erfolgen.

Hier und vorzugsweise werden die Saugsegmente 15a der Saugfläche 15 mit der Saugfläche 15 um die Hochachse 17 gedreht. Es ist vorzugsweise jedoch vorgesehen, dass, insbesondere auf der dem Hygienematerialstück 16 abgewandten Seite der Saugfläche 15, Saugsegmentzuführungen (nicht dargestellt) vorgesehen sind, die vakuumleitend mit den Saugsegmenten 15a der Saugfläche 15 verbunden sind. Es ist dann vorzugsweise vorgesehen, dass die Saugsegmentzuführungen nicht um die Hochachse 17 drehbar sind. Somit ändert sich die Zuordnung der Saugsegmentzuführungen zu den Saugsegmenten 15a während der Drehung der Saugfläche 15. Die Ansteuerung wird entsprechend angepasst.

Zusätzlich oder alternativ kann vorgesehen sein, dass die Saugsegmente 15a bei der Abgabe des Hygienematerialstücks 16, vorzugsweise entsprechend sequentiell, mit Druckluft beaufschlagt werden, damit das Vakuum schneller deaktiviert wird.

Hier und vorzugsweise sind mindestens vier, weiter vorzugsweise genau vier, Saugsegmente 15a und/oder Saugsegmentzuführungen vorgesehen.

Die Trommel 12 weist hier und vorzugsweise eine Antriebswelle 26 zur Erzeugung der Rotation der Trommel 12 um die Trommeldrehachse 11 auf. Die Antriebswelle 26 ist insbesondere zur Trommeldrehachse 11 konzentrisch. Die Antriebswelle 26 kann als Hohlwelle ausgestaltet sein, so dass der Unterdruck von einer Unterdruckquelle durch die Antriebswelle 26 zu der Saugfläche 15 geleitet werden kann. Dabei kann die Hohlwelle selbst den Unterdruck leiten oder einen Schlauch oder ähnliches umfassen. Vorzugsweise wird der Unterdruck über aus der Antriebswelle 26 herausführende Rohr- oder Schlauchverbindungen 27 zu der Saugfläche 15 und insbesondere zu den Saugsegmenten 15a geleitet.

Die Antriebswelle 26 kann auf einem, insbesondere zweiarmigen, Träger 28 gelagert sein. Die Drehvorrichtung 1, hier insbesondere der Träger 28, ist vorzugsweise freistehend.

Die Trommel 12 kann hier und vorzugsweise zwei beabstandete, winkelig, insbesondere rechtwinkelig, zur Trommeldrehachse 11 verlaufende Scheiben 13 aufweisen, mit denen der Teller 14 jeweils gekoppelt ist.

Wie bereits erwähnt, weist die Drehvorrichtung 1 vorzugsweise mehrere, insbesondere mindestens drei, weiter vorzugsweise mindestens sechs, noch weiter vorzugsweise genau acht, gleichartige Teller 14 auf. Ganz allgemein kann der Teller 14 derart mit der Trommel 12 gekoppelt sein, dass der Teller 14 ohne Schlupf mit der Trommel 12 rotiert.

Nach einer weiteren Lehre, der eigenständige Bedeutung zukommt, wird eine Fertigungsanlage 2 für Hygieneprodukte, insbesondere Erwachsenen- oder Babywindeln, vorgeschlagen. Die Fertigungsanlage 2 weist eine vorschlagsgemäße Drehvorrichtung 1 auf. Auf alle Ausführungen zu der vorschlagsgemäßen Drehvorrichtung 1 darf verwiesen werden. Diese Fertigungsanlage 2 kann hier und vorzugsweise mehrere Formate der Hygieneprodukte produzieren.

Die Fertigungsanlage 2 lässt sich am besten der Darstellung in Fig. 2 entnehmen. Hier und vorzugsweise schneidet die Fertigungsanlage 2 aus einer Endlos-Hygienematerial-Bahn 3 Hygienematerialstücke 16. Das Hygienematerial ist hier und vorzugsweise ein saugfähiges Material, so dass die Hygienematerialstücke 16 insbesondere Saugkerne sind. Weiterhin kann die Fertigungsanlage 2 aus einer Endlos-Trägermaterial-Bahn 29 Trägermaterialstücke schneiden, die hier und vorzugsweise Hüftbänder sind. Durch Verbinden der Hygienematerialstücke 16 und des Trägermaterials 9 fertigt die Fertigungsanlage 2 die Hygieneprodukte. Das Schneiden der Endlos-Trägermaterial-Bahn 29 kann vor oder nach dem Verbinden mit den Hygienematerialstücken 16 geschehen. Der Begriff "endlos" bedeutet hier lediglich, dass die Fertigungsanlage 2 so ausgelegt ist, dass die Länge der entsprechenden Bahn gegenüber der Länge eines entsprechenden einzelnen Stücks groß sein kann.

Die Fertigungsanlage 2 ist in Fig. 2 vom Fertigungsablauf her von rechts nach links dargestellt. Sie kann eine Zuführung für die Hygienematerial-Bahn 3 aufweisen, die hier ganz rechts dargestellt ist. Die Hygienematerial-Bahn 3 wird vorzugsweise über eine erste, mit einer ersten Geschwindigkeit v1 rotierende, Transportrolle 4 einer, mit einer zweiten Geschwindigkeit v2 rotierenden, Schneidrolle 6 zugeführt. Auf der Schneidrolle 6 wird die Hygienematerial-Bahn 3 durch eine Schneideinheit 7 in Hygienematerialstücke 16 geschnitten. Aufgrund der mit unterschiedlichen Geschwindigkeiten v1, v2 rotierenden Rollen 4, 6 entsteht vor der Schneideinheit 7 ein Schlupf der Hygienematerial-Bahn 3. In der Schneideinheit 7 auf der Schneidrolle 6 werden die Hygienematerialstücke 16 so beschleunigt, dass sie auf den Abstand zwischen den Tellern 14 der Drehvorrichtung 1 eingestellt sind. Dieser Abstand gibt gleichzeitig das maximal zu produzierende Format an Hygienematerialstücken 16 an. Die Hygienematerialstücke 16 werden im ersten Transferbereich 18 von der Schneidrolle 6 an die Drehvorrichtung 1 übergeben. Die Drehvorrichtung 1 dreht die Hygienematerialstücke 16 um die Hochachse 17 hier und vorzugsweise um 90°. Die Drehvorrichtung 1 kann die Hygienematerialstücke 16 dann an eine Repitch-Einheit 8 übergeben, die die Hygienematerialstücke 16 auf eine dritte Geschwindigkeit v3 beschleunigt oder abbremst. Die Fertigungsanlage 2 kann eine Trägerrolle 30 aufweisen, die mit der dritten Geschwindigkeit v3 rotiert und auf der das Trägermaterial 9, hier und vorzugsweise die Trägermaterial-Bahn 29 oder Trägermaterialstücke, geführt wird. Die Repitch-Einheit 8 übergibt die Hygienematerialstücke 16 an die Trägerrolle 30 und legt diese auf dem Trägermaterial 9 ab. Hier und vorzugsweise sind einige oder alle der Rollen ebenfalls mit Unterdruck ausgestattet, um das jeweilige Material zu halten. Dies kann ebenfalls für die Repitch-Einheit 8 gelten. Diese weist hier und vorzugsweise mehrere voneinander unabhängig rotierende Transferplatten 31 auf, um das Hygienematerial zu beschleunigen.

## Patentansprüche

1. Drehvorrichtung für eine Fertigungsanlage (2) für Hygieneprodukte, insbesondere Erwachsenen- oder Babywindeln,
wobei die Drehvorrichtung (1) eine um eine geometrische Trommeldrehachse (11) rotierende Trommel (12) und mindestens einen radial von der Trommeldrehachse (11) beabstandeten und mit der Trommel (12) gekoppelten Teller (14) aufweist,
wobei der Teller (14) eine Saugfläche (15) zum Transport von Hygienematerialstücken (16) umfasst, die das jeweilige Hygienematerialstück (16) während des Transports mittels eines Unterdrucks hält, wobei der Teller (14) eine radial zur Trommeldrehachse (11) verlaufende geometrische Hochachse (17) aufweist, wobei der Teller (14) das Hygienematerialstück (16) mit einer ersten Ausrichtung um die Hochachse (17) in einem ersten Transferbereich (18) aufnimmt, wobei der Teller (14) das Hygienematerialstück (16) entlang einer, insbesondere kreisförmigen, in Umfangsrichtung um die Trommeldrehachse (11) verlaufenden Soll-Transferbahn (19) von dem ersten Transferbereich (18) zu einem zweiten Transferbereich (20) transportiert,
wobei die Saugfläche (15) mit dem Hygienematerialstück (16) während des Transports um die Hochachse (17) gedreht wird,
wobei der Teller (14) das Hygienematerialstück (16) in dem zweiten Transferbereich (20) mit einer von der ersten Ausrichtung verschiedenen zweiten Ausrichtung um die Hochachse (17), insbesondere 90 Grad um die Hochachse (17) gedreht, abgibt,
**dadurch gekennzeichnet,**
**dass** der Teller (14) während der Aufnahme und/oder Abgabe eine nicht-lineare Ausgleichsbewegung relativ zur Trommel (12) mit radialem Bewegungsanteil durchführt.

2. Drehvorrichtung nach Anspruch 1, **dadurch gekennzeichnet, dass** die Ausgleichsbewegung des Tellers (14) zusätzlich einen Bewegungsanteil in Umfangsrichtung um die Trommeldrehachse (11) aufweist, und/oder, dass die Ausgleichsbewegung eine Rotationsbewegung des Tellers (14) um eine erste geometrische Rotationsachse (21) umfasst, weiter vorzugsweise, dass die Ausgleichsbewegung zusätzlich eine Rotation der ersten Rotationsachse (21) um eine zweite Rotationsachse (22) umfasst.

3. Drehvorrichtung nach Anspruch 1 oder 2, **dadurch gekennzeichnet, dass** der Teller (14) derart mit der Trommel (12) gekoppelt ist, dass der Fortschritt der Ausgleichsbewegung, insbesondere nur, von einem Drehwinkel der Trommel (12) um die Trommeldrehachse (11) abhängig ist.

4. Drehvorrichtung nach einem der vorhergehenden Ansprüche, **dadurch gekennzeichnet, dass** der Teller (14) mit der Trommel (12) ein Koppelgetriebe (23) bildet, vorzugsweise, dass das Koppelgetriebe (23) ein viergliedriges Koppelgetriebe (23), insbesondere eine Viergelenkkette, ist und/oder das Koppelgetriebe (23) ein ebenes Koppelgetriebe (23) ist, dessen Ebene vorzugsweise winklig, insbesondere rechtwinklig, zur Trommeldrehachse (11) ausgerichtet ist.

5. Drehvorrichtung nach einem der vorhergehenden Ansprüche, **dadurch gekennzeichnet, dass** der Teller (14) mittels eines Führungsgliedes (24) mit einer Kurvenscheibe (25) gekoppelt ist, dass die Ausgleichsbewegung durch die Kopplung des Tellers (14) über das Führungsglied (24) mit der Kurvenscheibe (25) verursacht wird, vorzugsweise, dass die Kurvenscheibe (25) um die Trommeldrehachse (11) angeordnet ist, weiter vorzugsweise, dass die Kurvenscheibe (25) nicht mit der Trommel (12) um die Trommeldrehachse (11) rotiert.

6. Drehvorrichtung nach einem der Ansprüche 1 bis 3, **dadurch gekennzeichnet, dass** die Ausgleichsbewegung eine Rotation des Tellers (14) um eine geometrische Rotationsachse ist, die vorzugsweise parallel, jedoch nicht koaxial, zur Trommeldrehachse (11) verläuft.

7. Drehvorrichtung nach einem der vorhergehenden Ansprüche, **dadurch gekennzeichnet, dass** die Aufnahme und/oder die Abgabe des Hygienematerialstücks (16) vollflächig mit einem konstanten Übergabeabstand erfolgt, und/oder, dass eine Geschwindigkeit des Hygienematerialstücks (16) während der Aufnahme und/oder Abgabe des Hygienematerialstücks (16) konstant bleibt.

8. Drehvorrichtung nach einem der vorhergehenden Ansprüche, **dadurch gekennzeichnet, dass** die Saugfläche (15) des Tellers (14) entlang seiner ersten und/oder zweiten Ausrichtung plan ist.

9. Drehvorrichtung nach einem der vorhergehenden Ansprüche, **dadurch gekennzeichnet, dass** ein in dem ersten Transferbereich (18) befindlicher Teil der Saugfläche (15) des Tellers (14) aufgrund der Ausgleichsbewegung während der Aufnahme radial innerhalb der Soll-Transferbahn (19) verbleibt, und/oder, dass ein in dem zweiten Transferbereich (20) befindlicher Teil der Saugfläche (15) des Tellers (14) aufgrund der Ausgleichsbewegung während der Abgabe radial innerhalb der Soll-Transferbahn (19) verbleibt, und/oder, dass die Saugfläche (15) entlang der ersten und/oder zweiten Ausrichtung nicht entlang der Soll-Transferbahn (19) verläuft.

10. Drehvorrichtung nach einem der vorhergehenden Ansprüche, **dadurch gekennzeichnet, dass** die Saugfläche (15) mehrere Saugsegmente (15a) umfasst, vorzugsweise, dass die Saugsegmente (15a) einzeln ansteuerbar sind, weiter vorzugsweise, dass die Saugsegmente (15a) abhängig von dem Drehwinkel der Trommel (12) und/oder einem Fortschritt der Ausgleichsbewegung angesteuert werden.

11. Drehvorrichtung nach einem der vorhergehenden Ansprüche, **dadurch gekennzeichnet, dass** die Trommel (12) eine Antriebswelle (26) zur Erzeugung der Rotation der Trommel (12) um die Trommeldrehachse (11) aufweist, vorzugsweise, dass die Antriebswelle (26) eine Hohlwelle ist und der Unterdruck von einer Unterdruckquelle durch die Antriebswelle (26) zu der Saugfläche (15), insbesondere den Saugsegmenten (15a), geleitet wird, und/oder, dass die Antriebswelle (26) auf einem, insbesondere zweiarmigen, Träger (28) gelagert ist.

12. Drehvorrichtung nach einem der vorhergehenden Ansprüche, **dadurch gekennzeichnet, dass** die Trommel (12) zwei beabstandete, winklig, insbesondere rechtwinklig, zur Trommeldrehachse (11) verlaufende Scheiben (13) aufweist, mit denen der Teller (14) jeweils gekoppelt ist.

13. Drehvorrichtung nach einem der vorhergehenden Ansprüche, **dadurch gekennzeichnet, dass** die Drehvorrichtung (1) mehrere, vorzugsweise mindestens drei, weiter vorzugsweise mindestens sechs, noch weiter vorzugsweise genau acht, gleichartige Teller (14) aufweist, und/oder, dass der Teller (14) derart mit der Trommel (12) gekoppelt ist, dass der Teller (14) ohne Schlupf mit der Trommel (12) rotiert.

14. Fertigungsanlage für Hygieneprodukte, insbesondere Erwachsenen- oder Babywindeln, **dadurch gekennzeichnet, dass** die Fertigungsanalage (2) eine Drehvorrichtung (1) nach einem der vorhergehenden Ansprüche aufweist.

15. Fertigungsanlage für Hygieneprodukte nach Anspruch 14, **dadurch gekennzeichnet, dass** die Fertigungsanlage (2) aus einer Endlos-Hygienematerial-Bahn (3) Hygienematerialstücke (16), insbesondere Saugkerne, schneidet, dass die Fertigungsanlage (2) aus einer Endlos-Trägermaterial-Bahn (29) Trägermaterialstücke, insbesondere Hüftbänder, schneidet und dass die Fertigungsanlage (2) durch Verbinden der Hygienematerialstücke (16) und des Trägermaterials (9) die Hygieneprodukte fertigt.

16. Fertigungsanlage für Hygieneprodukte nach Anspruch 14 oder 15, **dadurch gekennzeichnet, dass** die Fertigungsanlage (2) eine Zuführung für die Hygienematerial-Bahn (3) aufweist, dass die Hygienematerial-Bahn (3) über eine erste, mit einer ersten Geschwindigkeit (v1) rotierende Transportrolle (4) einer, mit einer zweiten Geschwindigkeit (v2) rotierenden Schneidrolle (6) zugeführt wird, dass auf der Schneidrolle (6) die Hygienematerial-Bahn (3) durch eine Schneideinheit (7) in Hygienematerialstücke (16) geschnitten wird, dass die Hygienematerialstücke (16) auf der Schneidrolle (6) auf die zweite Geschwindigkeit (v2) beschleunigt werden, dass die Drehvorrichtung (1) mit der zweiten Geschwindigkeit (v2) rotiert, dass die Hygienematerialstücke (16) im ersten Transferbereich (18) von der Schneidrolle (6) an die Drehvorrichtung (1) übergeben werden, dass die Drehvorrichtung (1) die Hygienematerialstücke (16) um die Hochachse (17) um 90 Grad dreht, dass die Drehvorrichtung (1) die Hygienematerialstücke (16) an eine Repitch-Einheit (8) übergibt, die die Hygienematerialstücke (16) auf eine dritte Geschwindigkeit (v3) beschleunigt oder abbremst, dass die Fertigungsanlage (2) eine Trägerrolle (30) aufweist, die mit der dritten Geschwindigkeit (v3) rotiert und auf der das Trägermaterial (9) geführt wird und dass die Repitch-Einheit (8) die Hygienematerialstücke (16) an die Trägerrolle (30) übergibt und auf dem Trägermaterial (9) ablegt.
